# EUROPÄISCHE PATENTSCHRIFT

(11) **EP 3 733 043 B1**
(45) Veröffentlichungstag und Bekanntmachung des Hinweises auf die Patenterteilung: **26.04.2023**
(21) Anmeldenummer: 20171798.0
(22) Anmeldetag: 28.04.2020
(51) Int. Cl.: A61B 1/00, A61B 1/267

(54) **ENDOSKOPISCHE VORRICHTUNG**
ENDOSCOPIC DEVICE
DISPOSITIF ENDOSCOPIQUE

(30) Priorität: 02.05.2019 DE 102019111347
(43) Veröffentlichungstag der Anmeldung: 04.11.2020
(73) Patentinhaber: Karl Storz SE & Co. KG, 78532 Tuttlingen (DE)
(72) Erfinder: Attinger, Jürg, 8260 Stein am Rhein (CH)

(56) Entgegenhaltungen:
- EP-A1- 2 000 078
- DE-A1- 19 916 047
- DE-U1- 20 003 797
- JP-A- 2002 172 118
- US-A1- 2003 220 545

## Beschreibung

### Stand der Technik

Die Erfindung betrifft eine endoskopische Vorrichtung gemäß dem Oberbegriff des Anspruch 1.

Aus der US 10,149,605 B2 ist bereits eine endoskopische Vorrichtung mit einem flexiblen Endoskopschaft und mit wenigstens einem den Endoskopschaft zugeordneten Knickschutz bekannt. Der Knickschutz weist einen zylindrisch ausgebildeten Grundkörper auf sowie zur wahlweisen Verbindung des Knickschutzes mit einem Tubus wenigstens einen Schnellverbinder, der über mehrere konzentrisch und entlang einer Zylinderachse des Grundkörpers versetzt zueinander angeordnete und sich zumindest im Wesentlichen senkrecht aus dem Grundkörper erstreckende Ringrippen verfügt.

Ebenso sind weitere Verbindungstechniken zum Verbinden von Tuben an einem Knickschutz oder des Knickschutzes an einem Endoskopschaft aus der US 2003/0220545, der DE 199 16 047 A1, der EP 2 000 078 A1, der DE 200 03 797 U1 sowie der JP 2002 172118 A bekannt.

Die Aufgabe der Erfindung besteht insbesondere darin, eine gattungsgemäße Vorrichtung mit verbesserten Eigenschaften hinsichtlich Sicherheit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer endoskopischen Vorrichtung mit wenigstens einem flexiblen Endoskopschaft und mit wenigstens einem den Endoskopschaft zugeordneten Knickschutz, welcher zu einer wahlweisen Verbindung des Knickschutzes mit einem Tubus eingerichtet ist, und welcher wenigstens einen, zumindest abschnittsweise konisch und/oder zylindrisch ausgebildeten Grundkörper, der eine Haupterstreckungsrichtung sowie eine Haupterstreckung entlang der Haupterstreckungsrichtung aufweist, umfasst, und der Knickschutz zur Verbindung mit dem Tubus wenigstens einen Schnellverbinder umfasst, welcher wenigstens eine zumindest im Wesentlichen senkrecht auf dem Grundkörper stehende Finne aufweist.

Es wird vorgeschlagen, dass die Finne eine Erstreckung aufweist, welche sich entlang der Haupterstreckungsrichtung des Grundkörpers über zumindest 50 %, insbesondere über zumindest 60%, vorzugsweise über zumindest 70 % und besonders bevorzugt über zumindest 80 %, der Haupterstreckung des Grundkörpers erstreckt.

Hierdurch kann vorteilhaft ein Halt des Tubus an dem Knickschutz verbessert werden, wodurch ein versehentliches ablösen vermieden und eine Sicherheit erhöht werden kann. Weiter vorteilhaft kann eine Sicherheit beim Entfernen des Tubus verbessert werden, da der Knickschutz aufgrund der vorliegenden Ausgestaltung nicht plötzlich seine Klemmkraft verliert, sondern gleichmäßig und langsam gelöst werden kann. Ferner vorteilhaft erzielt der Schnellverbinder auch eine sichere Verbindung auch mit Tuben welche beispielsweise aufgrund von Herstellungstoleranzen verschiedene Innendurchmesser aufweisen.

Unter einer "endoskopischen Vorrichtung" soll insbesondere ein, vorzugsweise funktionsfähiger Bestandteil, insbesondere eine Unterbaugruppe und/oder eine Konstruktions- und/oder eine Funktionskomponente eines Endoskops verstanden werden. Vorzugsweise kann die endoskopische Vorrichtung das Endoskop zumindest teilweise, vorzugsweise zumindest zu einem Großteil und besonders bevorzugt vollständig ausbilden. In etwa ist die endoskopische Vorrichtung dazu eingerichtet, zumindest teilweise und vorzugsweise zumindest zu einem Großteil in eine insbesondere künstliche und/oder natürliche Kavität, insbesondere Körperkavität, eingeführt zu werden und zwar insbesondere um diese zu begutachten. Bei der endoskopischen Vorrichtung kann es sich um eine medizinische und/oder industrielle endoskopische Vorrichtung handeln. Unter "eingerichtet" soll insbesondere speziell programmiert, ausgebildet, ausgelegt und/oder ausgestattet verstanden werden. Darunter, dass ein Bauteil zu einer bestimmten Funktion eingerichtet ist, soll insbesondere verstanden werden, dass das Bauteil diese bestimmte Funktion in zumindest einem Anwendungs- und/oder Betriebszustand erfüllt und/oder ausführt. Unter einem "Endoskopschaft" soll insbesondere ein länglicher Teil eines Endoskops verstanden werden, welcher in etwa dazu ausgebildet ist, in eine insbesondere künstliche und/oder natürliche Kavität, insbesondere Körperkavität, eingeführt zu werden. Unter einem "länglichen Teil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine größte Erstreckung des Bauteils senkrecht zu dessen Haupterstreckung, also insbesondere einem Durchmesser des Bauteils. Unter einer "Haupterstreckung" eines Bauteils, soll insbesondere dessen längste Erstreckung entlang dessen Haupterstreckungsrichtung verstanden werden. Unter einer "Haupterstreckungsrichtung" eines Bauteils soll insbesondere eine Richtung verstanden werden, welche parallel zu einer längsten Kante eines kleinsten gedachten Quaders verläuft, welcher das Bauteil gerade noch vollständig umschließt und welche vorzugsweise durch ein geometrischen Mittelpunkt und/oder durch einen Massenmittelpunkt des Bauteils verläuft. Der Endoskopschaft weist insbesondere einen distalen Endabschnitt und einen proximalen Endabschnitt auf. Unter einem "Endabschnitt" eines Bauteils soll insbesondere ein Abschnitt verstanden werden, welcher sich ausgehend von einem Ende des Bauteils zur Mitte des Bauteils hin, um höchstens 10 cm, vorzugsweise um höchstens 5 cm und besonders bevorzugt um höchstens 3 cm, erstreckt. Unter einem "distalen Endabschnitt" eines Bauteils soll insbesondere ein Endabschnitt verstanden werden, welcher sich ausgehend von einem distalen Ende des Bauteils in proximaler Richtung erstreckt. Unter einem "proximalen Endabschnitt" eines Bauteils soll insbesondere ein Endabschnitt verstanden werden, welcher sich ausgehend von einem proximalen Ende des Bauteils in distaler Richtung erstreckt. Unter "distal" soll insbesondere bei einer Bedienung einem Patienten zugewandt und/oder einem Bediener abgewandt verstanden werden. Insbesondere ist proximal das Gegenteil von distal. Unter "proximal" soll insbesondere bei einer Bedienung einem Patienten abgewandt und/oder einem Bediener zugewandt verstanden werden. Ferner weist die endoskopische Vorrichtung insbesondere zumindest eine Handhabe auf. Die Handhabe ist in etwa am proximalen Endabschnitt des Endoskopschafts angeordnet. Die Handhabe ist insbesondere für eine manuelle Bedienung der endoskopischen Vorrichtung ausgebildet. Die Handhabe umfasst in etwa zumindest einen Handgriff und/oder zumindest ein Bedienelement, wie beispielsweise ein Schalter, Knopf oder dergleichen, welches vorzugsweise am Handgriff angeordnet ist. Unter einem "Knickschutz" soll insbesondere ein Bauteil verstanden werden, welche dazu eingerichtet ist, ein Abknicken des Endoskopschafts zu vermeiden und zwar insbesondere am proximalen Endabschnitt des Endoskopschafts, vorzugsweise im Übergangsbereich des Endoskopschafts zur Handhabe. Insbesondere ist der Knickschutz als Ganzes betrachtet frei von einer Drehsymmetrie. Vorzugsweise weist der Knickschutz eine Spiegelsymmetrie auf, wobei eine Spiegelebene der Spiegelsymmetrie wenigstens einer Haupterstreckungsebene einer Finne entspricht. Unter einer "Haupterstreckungsebene" eines Bauteils soll insbesondere eine Ebene verstanden werden, welche parallel zu einer größten Seitenfläche eines kleinsten gedachten Quaders ist, welcher das Bauteil gerade noch vollständig umschließt, und insbesondere durch den Mittelpunkt des Quaders verläuft. Bei dem Tubus handelt es sich insbesondere um einen Beatmungstubus oder Endotrachealtubus, welcher dazu eingerichtet ist, in eine Luftröhre eingeführt zu werden. Der Tubus weist insbesondere einen Kanal auf, welcher dazu eingerichtet ist, den Endoskopschaft aufzunehmen. Unter einem "Schnellverbinder" soll insbesondere ein Bauteil verstanden werden, welches dazu eingerichtet ist, wenigstens zwei Bauteile lösbar und werkzeuglos miteinander zu verbinden. Vorzugsweise ist der Schnellverbinder zur Ausbildung einer kraft- und/oder formschlüssigen Verbindung vorgesehen, wie beispielsweise einer Klemmverbindung. Unter "zumindest im Wesentlichen senkrecht" soll insbesondere eine Ausrichtung einer Richtung relativ zu einer Bezugsrichtung, insbesondere in einer Ebene, verstanden werden, wobei die Richtung und die Bezugsrichtung einen Winkel von 90°, insbesondere unter Berücksichtigung einer maximalen Abweichung von kleiner als 8°, vorteilhaft von kleiner als 5° und besonders vorteilhaft von kleiner als 2° einschließt.

Es wäre denkbar, dass sich die Finne gewindeartig um den Grundkörper windet. Um insbesondere jedoch ein sicheres und leicht gängiges Abziehen des Tubus zu erzielen wird vorgeschlagen, dass eine Haupterstreckungsebene der Finne zumindest im Wesentlichen parallel zu Haupterstreckungsrichtung des Grundkörpers ausgerichtet ist. Unter "zumindest im Wesentlichen parallel" soll insbesondere eine Ausrichtung einer Richtung relativ zu einer Bezugsrichtung, insbesondere in einer Ebene, verstanden werden, wobei die Richtung und die Bezugsrichtung einen Winkel von 0°, insbesondere unter Berücksichtigung einer maximalen Abweichung von kleiner als 8°, vorteilhaft von kleiner als 5° und besonders vorteilhaft von kleiner als 2° einschließt.

Ferner wird vorgeschlagen, dass die Finne wenigstens eine dem Grundkörper abgewandte Außenkante aufweist, wobei ein Winkel der Außenkante relativ zur Haupterstreckungsrichtung entlang der Haupterstreckung des Grundkörpers, insbesondere in proximaler Richtung hin, abnimmt. Es kann vorteilhaft eine Sicherheit weiter verbessert werden, da ein Tubus entlang der Finne bei einem Einführvorgang zentriert werden kann. Weiter vorteilhaft kann ein leicht gängiges Verbinden eines Tubus mit dem Knickschutz erreicht werden. Der Winkel kann dabei kontinuierlich und/oder Stufenweise abnehmen. Insbesondere weist die Finne verschiedene Abschnitte auf, in welchen der Winkel stufenweise abnimmt. Insbesondere beträgt der Winkel in einem ersten Abschnitt der Finne höchstens 80°, in wenigstens einem zweiten Abschnitt der Finne höchstens 10° und/oder in wenigstens einem dritten Abschnitt der Finne höchstens 5°.

Es wird vorgeschlagen, dass die Finne dazu eingerichtet ist, sich bei einer Kontaktierung, insbesondere mit dem Tubus, seitlich zu verbiegen. Es kann vorteilhaft eine Sicherheit weiter verbessert werden. Insbesondere kann ein vorteilhaftes Verklemmen eines Tubus am Knickschutz erzielt werden. Darunter, dass die Finne dazu eingerichtet ist, sich seitlich zu verbergen, soll insbesondere versstanden werden, dass sich die Finnen relativ zu ihrer Haupterstreckungsebene betrachtet aus dieser heraus konvex und/oder konkav verformt.

Es wird vorgeschlagen, dass die Finne eine maximale Höhe relativ zum Grundkörper aufweist, welche wenigstens einer doppelten Dicke der Finne entspricht. Es kann vorteilhaft eine Sicherheit weiter verbessert werden. Insbesondere kann ein besonders leichtes Auslenken der Finne erzielt werden. Die Dicke ist dabei insbesondere senkrecht zur Haupterstreckungsebene der Finne gemessen.

Es wird vorgeschlagen, dass die Finne wenigstens teilweise, vorzugsweise zumindest zu einem Großteil und besonders bevorzugt vollständig, elastisch ausgebildet ist. Es kann vorteilhaft eine Sicherheit bei einer Montage und/oder Demontage des Tubus weiter verbessert werden. Unter einem "elastischen Bauteil" soll insbesondere ein Bauteil verstanden werden, welches bei einer Verformung aus einer Grundstellung heraus wieder von alleine in die Grundstellung strebt.

Es wird weiter vorgeschlagen, dass die Finne wenigstens teilweise, vorzugsweise zumindest zu einem Großteil und besonders bevorzugt vollständig, aus einem Material besteht, welches einen Shorewert von wenigstens 40, insbesondere wenigstens 50, vorzugsweise wenigstens 60, und/oder höchstens 90, insbesondere höchstens 80, vorzugsweise höchstens 70 aufweist. Hierdurch kann vorteilhaft eine Sicherheit weiter verbessert werden. Insbesondere kann hierdurch ein guter Kompromiss zwischen leichter Montage bzw. Demontage und sicherem Halt des Tubus an dem Knickschutz gefunden werden. Unter dem Ausdruck "zumindest zu einem Großteil" soll dabei insbesondere zumindest zu 55 %, vorzugsweiser zumindest zu 65 %, bevorzugt zumindest zu 75 %, besonders bevorzugt zumindest zu 85 % und ganz besonders bevorzugt zumindest zu 95 %, sowie vorteilhaft vollständig verstanden werden und zwar insbesondere mit Bezug auf ein Volumen und/oder eine Masse eines Bauteils. Besonders bevorzugt beträgt der Shorewert zumindest im Wesentlichen 65. Unter "zumindest im Wesentlichen" soll insbesondere eine maximale Abweichung von höchstens 10 %, vorzugsweise von höchstens 5 % und besonders bevorzugt von höchstens 2 % umfassend verstanden werden.

Es wird ferner vorgeschlagen, dass die Finne wenigstens teilweise, vorzugsweise zumindest zu einem Großteil und besonders bevorzugt vollständig, aus einem Material, insbesondere das vorbenannte Material besteht, bei welchem es sich um ein thermoplastisches Elastomer handelt. Insbesondere kann eine Sicherheit weiter verbessert werden. Das thermoplastische Elastomer ist vorzugsweise das thermoplastische Elastomer, welches unter dem Markennamen Mediprene bekannt ist.

Des Weiteren wir vorgeschlagen, dass der Grundkörper zumindest einen konischen Abschnitt und wenigstens einen zylindrischen Abschnitt aufweist, Insbesondere kann eine Sicherheit weiter verbessert werden. Insbesondere kann vorteilhaft eine Klemmwirkung der Finne zwischen Tubus und Grundkörper verbessert werden. Insbesondere kann der Grundkörper zumindest zwei zylindrische Abschnitte aufweisen, welche vorzugsweise durch wenigstens einen konischen Abschnitt miteinander verbunden sind.

Der Grundkörper weist wenigstens einen Abschnitt, insbesondere einen zylindrischen Abschnitt auf, in welchem der Grundkörper senkrecht zu dessen Haupterstreckungsrichtung geschnitten wenigstens eine kreisringförmige Form Querschnitt aufweist. Um insbesondere einen sicheren Halt zu ermöglichen und eine Orientierung des Tubus relativ zum Knickschutz klar zu definieren, wird vorgeschlagen, dass der Grundkörper senkrecht zu dessen Haupterstreckungsrichtung geschnitten wenigstens abschnittsweise einen von einer kreisringförmigen Form abweichenden Querschnitt aufweist. Insbesondere weist wenigstens ein konischer Abschnitt des Grundkörpers senkrecht zu dessen Haupterstreckungsrichtung geschnitten wenigstens einen von einer Kreisringform abweichenden Querschnitt auf. Vorzugsweise weist der Grundkörper im konischen Abschnitt senkrecht zu dessen Haupterstreckungsrichtung geschnitten wenigstens einen ovalringförmigen Querschnitt auf. Vorzugsweise ist der Grundkörper frei von einer Drehsymmetrie. Denkbar ist, dass einzelne Abschnitte, wie beispielsweise ein konischer Abschnitt und/oder ein kreisringförmiger Abschnitt eine Drehsymmetrie aufweisen, diese jedoch entlang ihrer jeweiligen Drehsymmetrieachsen zueinander versetzt angeordnet sind, sodass der die Abschnitte aufweisende Grundkörper insgesamt von einer Drehsymmetrie frei ist. Alternativ könnte der Grundkörper eine endliche Drehsymmetrie auf. Vorzugsweise weist der Grundkörper eine n-zählige Drehsymmetrie auf, wobei n eine von unendlich verschiedener Zahl ist. Besonders bevorzugt ist n eine gerade Zahl und beträgt ganz besonders bevorzugt den Wert zwei.

Es wird vorgeschlagen, dass die Finne einstückig mit dem Grundkörper ausgebildet ist. Hierdurch kann eine Sicherheit weiter verbessert werden. Insbesondere kann vermieden werden, dass sich einzelne Finnen vom Grundkörper bei einer Benutzung lösen und zu einem ungewollten aufheben der Verbindung des Tubus mit dem Knickschutz führen könnten. Unter "einstückig" soll insbesondere zumindest stoffschlüssig verbunden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling.

Es wird vorgeschlagen, dass der Schnellverbinder wenigstens eine weitere Finne aufweist, welche im Wesentlichen identisch zu der Finne ausgebildet ist. Hierdurch kann vorteilhaft eine Sicherheit weiter verbessert werden. Weiter vorteilhaft kann eine Klemmkraft erhöht werden. Die Finne und die weitere Finne sind insbesondere zueinander in Umfangsrichtung um den Grundkörper beabstandet angeordnet. Insbesondere weist der Schnellverbinder eine Anzahl von m Finnen auf, wobei insbesondere gelten soll m= n+1 und somit vorzugsweise eine ungerade Zahl ist. Besonders bevorzugt beträgt der Wert der Zahl m drei.

Es wird vorgeschlagen, dass die Finne und die weitere Finne um eine Drehsymmetrieachse relativ zueinander Drehsymmetrisch an dem Grundkörper angeordnet sind. Hierdurch kann vorteilhaft eine Sicherheit weiter erhöht werden. Weiter vorteilhaft kann eine symmetrische Anordnung des Tubus um den Knickschutz erzielt werden, wodurch insbesondere ein Aufziehen des Tubus auf den Knickschutz sicherer gestaltet werden kann. Insbesondere kann durch diese Anordnung eine gleichmäßige Verteilung von Klemmkräften erzielt werden. Insbesondere weist die Drehsymmetrie den Wert I auf, welcher vorzugsweise von dem Wert n verschieden ist und ganz besonders bevorzugt gleich dem Wert m ist. Insbesondere entspricht eine Drehsymmetrieachse des ringförmigen Abschnitts des Grundkörpers der Drehsymmetrieachse der Finnen.

Es wird vorgeschlagen, dass die Drehsymmetrieachse der Finnen verschieden ist von einer Haupterstreckungsrichtung des Grundkörpers. Hierdurch lässt sich vorteilhaft eine Sicherheit weiter erhöhen. Weiter vorteilhaft kann eine Orientierung des Knickschutzes relativ zum Tubus festgelegt werden, wodurch sich eine poka-yoke artige Zuordnung erzielen lässt. Insbesondere ist eine Drehsymmetrieachse, um welche die Finnen drehsymmetrisch angeordnet sind parallel zur Haupterstreckungsrichtung des Grundkörpers. Vorzugsweise ist die Drehsymmetrieachse, um welche die Finnen drehsymmetrisch angeordnet sind, parallel versetzt zur Haupterstreckungsrichtung des Grundkörpers angeordnet.

Um die Sicherheit weiter zu erhöhen wird vorgeschlagen, dass die endoskopische Vorrichtung den Tubus umfasst. Insbesondere ist der Tubus korrespondierend zu dem Endoskopschaft, dem Knickschutz und/oder dem Schnellverbinder ausgebildet.

Ferner wird ein Endoskop mit wenigstens der endoskopischen Vorrichtung beansprucht. Hierdurch kann ein Endoskop mit verbesserten Eigenschaften bezüglich einer Sicherheit bereitgestellt werden.

Ferner wird ein Verfahren zur Montage und/oder Demontage der endoskopischen Vorrichtung beschrieben, welches nicht Teil des beanspruchten Gegenstands ist. Hierdurch kann sicher eine Montage und/oder Demontagevorgang erzielt werden.

Ferner wird ein Verfahren zur Herstellung der endoskopischen Vorrichtung beschrieben, welches nicht Teil des beanspruchten Gegenstands ist.

Hierdurch kann eine sichere endoskopische Vorrichtung bereitgestellt werden. Insbesondere kann vermieden werden, dass sich einzelne Komponenten der endoskopischen Vorrichtung, wie beispielsweise die Finne des Knickschutzes, lösen können und eine sicher Verbindung gefährden.

Die erfindungsgemäße endoskopische Vorrichtung und/oder das Verfahren sollen/soll hierbei nicht auf die oben beschriebene Anwendung und Ausführungsform beschränkt sein. Insbesondere kann die erfindungsgemäße endoskopische Vorrichtung und/oder das Verfahren zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf die Vorrichtung beschriebenen Merkmale und Eigenschaften, aber auch Verfahrensweisen sinngemäß übertragbar und im Sinne der Erfindung einsetzbar und als mitoffenbart gelten. Gleiches gilt auch in umgekehrter Richtung. Das bedeutet, dass auch in Bezug auf das Verfahren genannte bauliche, also vorrichtungsgemäße Merkmale im Rahmen der Vorrichtungsansprüche berücksichtigt, beansprucht und ebenfalls zur Offenbarung gezählt werden können.

Falls von einem bestimmten Bauteil mehr als ein Exemplar vorhanden ist, ist nur eines davon in den Figuren und in der Beschreibung mit einem Bezugszeichen versehen. Die Beschreibung dieses Exemplars kann entsprechend auf die anderen Exemplare von dem Bauteil übertragen werden.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung eines Endoskops mit einer endoskopischen Vorrichtung in einer perspektivischen Ansicht,
- Fig. 2: eine schematische Darstellung eines Teils der endoskopischen Vorrichtung mit einem Knickschutz in einer perspektivischen Ansicht,
- Fig. 3: eine schematische Darstellung des Knickschutzes in einer Seitenansicht,
- Fig. 4: eine schematische Darstellung des Knickschutzes in einer Rückansicht,
- Fig. 5: eine schematische Darstellung des Knickschutzes in einer Frontansicht,
- Fig. 6: eine schematische Darstellung des Knickschutzes in einer Schnittansicht,
- Fig. 7: eine schematische Darstellung des Knickschutzes in einer weiteren Schnittansicht,
- Fig. 8: eine schematische Darstellung des Knickschutzes in einer zusätzlichen Schnittansicht,
- Fig. 9: einen schematischen Ablaufplan eines beispielhaften Verfahrens zur Herstellung der endoskopischen Vorrichtung und
- Fig. 10: einen schematischen Ablaufplan eines beispielhaften Verfahrens zur Montag und/oder Demontage der endoskopische Vorrichtung.

### Beschreibung der Ausführungsbeispiele

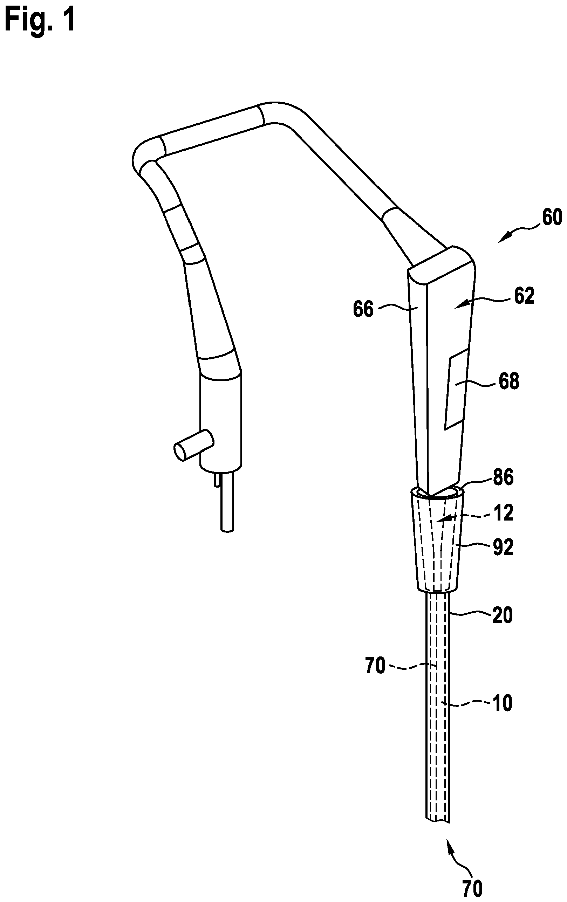

Fig. 1 zeigt eine schematische Darstellung eines Endoskops 60 mit einer endoskopischen Vorrichtung in einer perspektivischen Ansicht. Im vorliegenden Fall bildet die endoskopische Vorrichtung das Endoskop 60 vollständig aus. Alternativ könnte die endoskopische Vorrichtung auch nur ein Bestandteil, eine Baugruppe des Endoskops 60 oder dergleichen sein.

Die endoskopische Vorrichtung weist eine Handhabe 62 auf. Die Handhabe 62 weist zumindest einen Handgriff 64 auf. Die Handhabe 62 weist ein Gehäuse 66 auf. Das Gehäuse 66 dient zur Anordnung weiterer Bauteile der endoskopischen Vorrichtung. Beispielsweise ist eine Ablenkungsmechanik zum Ablenken des Endoskopschafts 10 in dem Gehäuse 66 angeordnet. Die Handhabe 62 weist ein Bedienelement 68 auf. Das Bedienelement 68 ist zu einer Bedienung einer Ablenkung eines Endoskopschafts 10 eingerichtet. Das Bedienelement 68 ist mit der Ablenkmechanik wirkverbunden.

Die endoskopische Vorrichtung weist den Endoskopschaft 10 auf. Der Endoskopschaft 10 ist flexibel ausgebildet. Ferner ist der Endoskopschaft 10 wenigstens abschnittsweise ablenkbar. Der Endoskopschaft 10 ist mittels der Ablenkmechanik ablenkbar. Der Endoskopschaft 10 ist über die Ablenkmechanik mit dem Bedienelement 68 wirkverbunden.

Die endoskopische Vorrichtung weist einen Tubus 20 auf. Bei dem Tubus 20 handelt es sich um einen Beatmungstubus oder einen Endotrachealtubus. Der Tubus 20 ist dazu eingerichtet, in eine Luftröhre eingeführt zu werden. Der Tubus 20 weist einen Kanal 70 auf, welcher dazu eingerichtet ist, den Endoskopschaft 10 aufzunehmen. Der Endoskopschaft 10 ist wenigstens teilweise in den Tubus 20 einführbar. Der Tubus 20 weist einen proximalen Endabschnitt 92 auf. Der proximale Endabschnitt 92 ist als ein Hohlzylinder ausgebildet. Der proximale Endabschnitt 92 des Tubus 20 ist zu einer Aufnahme des Knickschutzes 12 eingerichtet.

Die endoskopische Vorrichtung weist wenigstens einen Knickschutz 12 auf. Der Knickschutz 12 ist dem Endoskopschaft 10 zugeordnet. Der Knickschutz 12 ist dazu eingerichtet, ein Abknicken und insbesondere eine damit verbundene Beschädigung des Endoskopschafts 10 zu vermeiden.

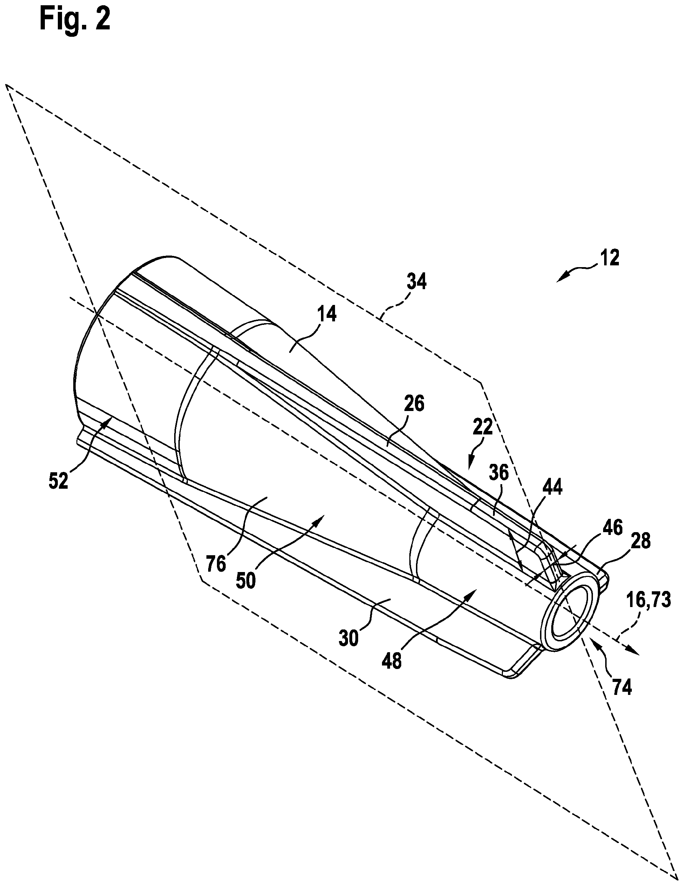

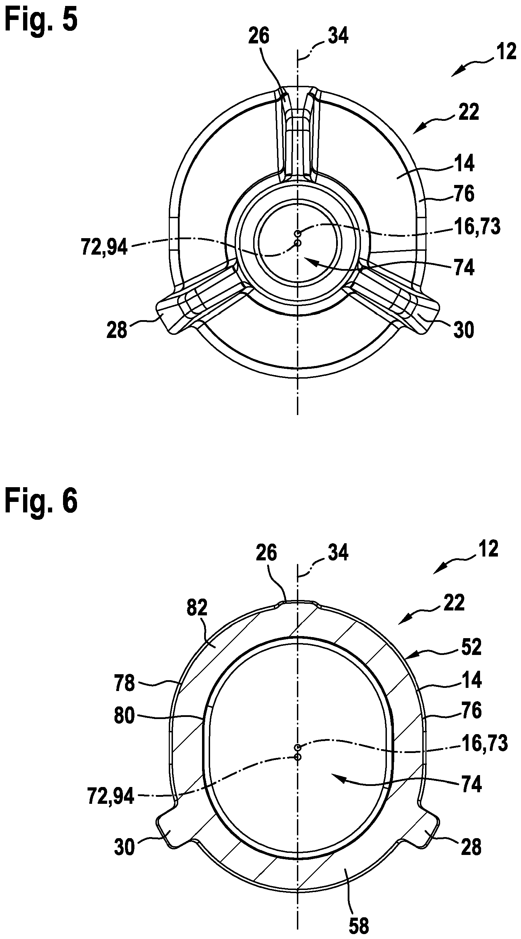

Figs. 2 bis 5 zeigen schematische Darstellung des Knickschutzes 12 in verschiedenen perspektivischen Ansichten. Ferner zeigen die Figuren 6 bis 8 eine schematische Darstellung des Knickschutzes 12 in verschiedenen Schnittansichten.

Der Knickschutz 12 weist einen Grundkörper 14 auf. Der Grundkörper 14 ist frei von einer Drehsymmetrie. Der Grundkörper 14 weist eine Haupterstreckungsrichtung 16 auf. Der Grundkörper 14 weist einer Haupterstreckung 18 entlang der Haupterstreckungsrichtung 16 auf.

Der Grundkörper 14 weist ferner eine Ausnehmung 74 auf. Die Ausnehmung 74 ist zur Aufnahme des Endoskopschafts 10 eingerichtet. Ein geringster Innendurchmesser der Ausnehmung 74 entspricht zumindest einem größten Außendurchmesser des Endoskopschafts 10.

Der Grundkörper 14 weist einen Mantel 76 auf. Der Mantel 76 erstreckt sich auf die Haupterstreckung 18 des Grundkörpers 14. Der Mantel 76 weist eine Außenfläche 78 auf. Ferner weist der Mantel 76 eine Innenfläche 80 auf. Der Mantel 76 weist eine Wandung 82 auf. Die Wandung 82 ist von der Innenfläche 80 und der Außenfläche 78 begrenzt. Die Innenfläche 80 begrenzt die Ausnehmung 74. Die Wandung 82 weist eine Wandstärke auf. Die Wandstärke der Wandung 80 ist entlang einer Haupterstreckung 18 des Grundkörpers 14 im Wesentlichen konstant.

Der Grundkörper 14 ist wenigstens teilweise elastisch ausgebildet. Im vorliegenden Fall ist der Grundkörper 14 zumindest zu einem Großteil einstückig ausgebildet. Der Grundkörper 14 weist einen Shorewert von wenigstens 40 auf. Ferner weist der Grundkörper 14 einen Shorewert von höchstens 80 aufweist. Im vorliegenden Fall weist der Grundkörper 14 weist einen Shorewert von zumindest im Wesentlichen 62 auf.

Der Grundkörper 14 besteht zumindest teilweise aus einem medizinischen Kunststoff. Im vorliegenden Fall besteht der Grundkörper zumindest zu einem Großteil aus einem medizinischen Kunststoff. Der medizinische Kunststoff ist dazu eingerichtet, Autoklavierprozesse zu wiederstehen. Bei dem Kunststoff handelt es sich um ein thermoplastisches Elastomer. Im vorliegenden Fall ist das thermoplastische Elastomer unter dem Markennamen Mediprene bekannt.

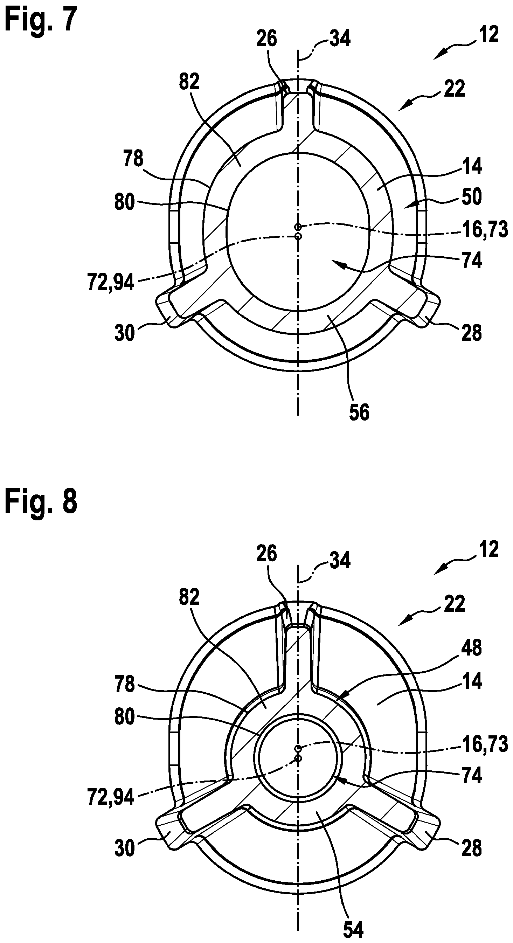

Der Grundkörper 14 weist wenigstens einen zylindrischen Abschnitt 48 auf (vgl. Fig. 8). Der zylindrische Abschnitt 48 ist ein distaler Abschnitt des Grundkörpers 14. Die Ausnehmung 74 erstreckt sich zumindest durch den zylindrischen Abschnitt 48. Der zylindrische Abschnitt 48 weist einen Außendurchmesser auf. Ferner weist der zylindrische Abschnitt 48 einen Innendurchmesser auf. Der Innendurchmesser korrespondiert zu einem Außendurchmesser des Endoskopschafts 10. Der zylindrische Abschnitt weist eine Drehsymmetrieachse 72 auf. Die Haupterstreckungsrichtung 16 des Grundkörpers ist zumindest parallel zur Drehsymmetrieachse 72 versetzt.

Zudem weist der Grundkörper 14 wenigstens einen weiteren zylindrischen Abschnitt 52 auf. Der weitere zylindrische Abschnitt 52 ist ein proximaler Abschnitt des Grundkörpers 14. Die Ausnehmung 74 erstreckt sich durch den weiteren zylindrischen Abschnitt 52. Im Bereich des zylindrischen Abschnitts 52 weist der Grundkörper 14 einen kreisringförmigen Querschnitt 54 auf (vgl. Fig. 8).

Der weitere zylindrische Abschnitt 52 weist einen weiteren Außendurchmesser auf. Der weitere Außendurchmesser des zylindrischen Abschnitts 52 ist größer als der Außendurchmesser des zylindrischen Abschnitts 48. Ferner weist er zylindrische Abschnitt 52 einen weiteren Innendurchmesser auf. Der weitere Innendurchmesser entspricht zumindest im Wesentlichen einem Außendurchmesser der Handhabe 62. Der weitere Innendurchmesser des weiteren zylindrischen Abschnitts 52 ist größer als der Innendurchmesser des zylindrischen Abschnitts 48. Ferner ist der weitere Innendurchmesser des Weiteren zylindrischen Abschnitts 52 größer als der Außendurchmesser des zylindrischen Abschnitts 48. Im Bereich des weitere zylindrischen Abschnitts 52 weist der Grundkörper 14 einen ovalringförmigen Querschnitt 58 auf (vgl. Fig. 6). Der weitere zylindrische Abschnitt 52 weist eine Drehsymmetrieachse 73 auf. Die Haupterstreckungsrichtung 16 des Grundkörpers ist identisch mit der Drehsymmetrieachse 73.

Ferner weist der Grundkörper wenigstens einen konischen Abschnitt 50 auf. Der konische Abschnitt 50 ist ein mittlerer Abschnitt des Grundkörpers 14. Die Ausnehmung 74 erstreckt sich durch den konischen Abschnitt 50. Der konische Abschnitt 50 ist zwischen dem zylindrischen Abschnitt 48und dem weiteren zylindrischen Abschnitt 52 angeordnet. Der konische Abschnitt 50 verbindet den zylindrischen Abschnitt 48 und den weiteren zylindrischen Abschnitt 52 miteinander. Der konische Abschnitt 50 weist einen Außendurchmesser auf. Der Außendurchmesser des konischen Abschnitts vergrößert sich entlang einer Haupterstreckungsrichtung des Grundkörpers von dem Außendurchmesser des zylindrischen Abschnitts 48 hin zum weiteren Außenradius des weiteren zylindrischen Abschnitts 52. Der konische Abschnitt 50 weist einen Innenradius auf. Der Innenradius deskonischen Abschnitts 50 vergrößert sich von dem Innenradius des zylindrischen Abschnitts 48 hin zum weiteren Innenradius des weiteren zylindrischen Abschnitts 48. Die Vergrößerung der Durchmesser erfolgt im vorliegenden Fall kontinuierlich. Alternativ könnte die Vergrößerung der Durchmesser schrittweise erfolgen. Im Bereich des konischen Abschnitts 50 weist der Grundkörper 14 einen ovalringförmigen Querschnitt 56 auf (vgl. Fig. 6).

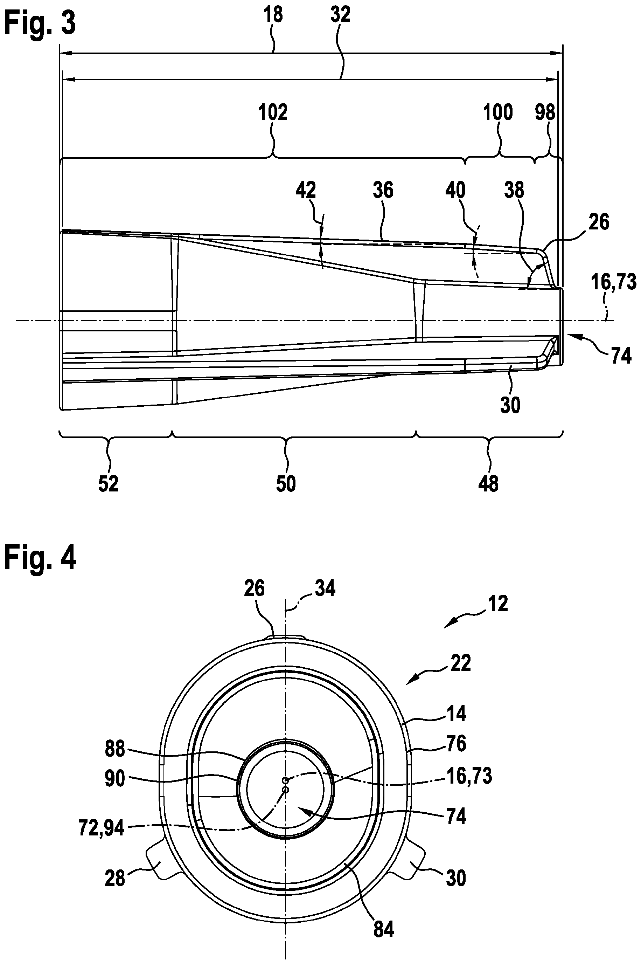

Zu einer Verbindung des Knickschutzes 12 mit der Handhabe 62, weist der Knickschutz 12 wenigstens ein Verbindungselement 84 auf (vgl. Fig. 4). Das Verbindungselement 84 ist am Grundkörper 14 angeordnet. Das Verbindungselement 84 ist im Abschnitt 52 angeordnet. Das Verbindungselement 84 ist an der Innenfläche 80 angeordnet. Im vorliegenden Fall ist das Verbindungselement 84 als eine Ringlippe ausgebildet. Das Verbindungselement 84 ist einstückig mit dem Grundkörper 14 ausgebildet. Ist der Knickschutz 12 bis zur Handhabe 62 hin auf den Endoskopschaft 10 aufgeschoben, stellt das Verbindungselement 84 eine Verbindung des Knickschutzes 12 mit der Handhabe 62 her. Die Handhabe 62 weist ein zu dem Verbindungselement 84 korrespondierendes Verbindungselement 86 auf, welches das Verbindungselement 84 bei einer Verbindung hintergreift.

Zu einer Arretierung des Knickschutzes 12 mit dem Endoskopschaft 10, weist der Knickschutz 12 wenigstens ein Arretierelement 88 auf (vgl. Fig. 4). Im vorliegenden Fall weist der Knickschutz wenigstens ein weiteres Arretierelement 90 auf, welches zu dem Arretierelement 88 beabstandet angeordnet ist. Im Folgenden ist nur ein Arretierelement 88 näher beschrieben. Die Beschreibung ist auf das weitere Arretierelement 90 übertragbar. Das Arretierelement 88 ist am Grundkörper 14 angeordnet. Das Arretierelement 88 ist im Abschnitt 48 angeordnet. Das Arretierelement 88 ist an der Innenfläche 80 angeordnet. Im vorliegenden Fall ist das Arretierelement 88 als eine Ringlippe ausgebildet. Das Arretierelement 88 ist einstückig mit dem Grundkörper 14 ausgebildet. Wird der Knickschutz 12 auf den Endoskopschaft 10 aufgeschoben, verklemmt sich das Arretierelement 88 mit dem Endoskopschaft 10.

Der Knickschutz 12 weist zu einer lösbaren Verbindung mit dem Tubus 20 wenigstens einen Schnellverbinder 22 auf. Der Schnellverbinder 22 ist zu einer form- und/oder kraftschlüssigen Verbindung mit dem Tubus 20 eingerichtet. Der Schnellverbinder 22 weist wenigstens eine Finne 26 auf. Die Finne 26 ist zumindest im Wesentlichen senkrecht auf dem Grundkörper 14 stehend angeordnet. Die Finne 26 steht senkrecht auf dem Mantel 76 des Grundkörpers 14. Die Finne 26 ist dazu eingerichtet, sich bei einer Kontaktierung durch den Tubus 20 seitlich zu verbiegen.

Die Finne 26 weist eine Erstreckung 32 entlang der Haupterstreckungsrichtung 16 des Grundkörpers 14, welche sich wenigstens über 50 % der Haupterstreckung 18 des Grundkörpers 14 erstreckt (vgl. Fig. 3). Im vorliegenden Fall erstreckt sich die Erstreckung 18 der Finne 26 sogar über wenigstens 80 % der Haupterstreckung 18 des Grundkörpers 14.

Ferner ist eine Haupterstreckungsebene 34 der Finne 26 zumindest im Wesentlichen parallel zu Haupterstreckungsrichtung 16 des Grundkörpers 14 ausgerichtet. Genauer gesagt liegt die Haupterstreckungsrichtung 16 des Grundkörpers 14 in der Haupterstreckungsebene 34 der Finne 26.

Die Finne 26 weist wenigstens eine dem Grundkörper 14 abgewandte Außenkante 36 auf. Ein Winkel 38, 40, 42 der Außenkante 36 relativ zur Haupterstreckungsrichtung 16 nimmt entlang der Haupterstreckung 18 des Grundkörpers 14 ab und zwar insbesondere in proximaler Richtung. Im vorliegenden Fall nimmt der Winkel 38, 40, 42 stufenweise ab. Alternativ könnte der Winkel 38, 40, 42 auch kontinuierlich abnehmen.

Die Finne 26 weist wenigstens einen ersten Abschnitt 98 auf. Der erste Abschnitt 98 erstreckt sich innerhalb eines Bereichs des zylindrischen Abschnitts 48 des Grundkörpers 14. Im ersten Abschnitt 98 weist die Außenkante 36 einen Winkel 38 relativ zur Haupterstreckungsrichtung 16 von höchstens 80° auf. Ferner weist die Finne 26 einen zweiten Abschnitt 100 auf. Der zweite Abschnitt 100 erstreckt sich in einem Bereich des zylindrischen Abschnitts 48 des Grundkörpers 14. Im zweiten Abschnitt 100 weist die Außenkante 36 der Finne 26 einen Winkel 40 relativ zur Haupterstreckungsrichtung 16 von höchstens 10° auf. Ferner weist die Finne 26 einen dritten Abschnitt 102 auf. Der dritte Abschnitt 102 erstreckt sich über einen Bereich des zylindrischen Abschnitts 48, des konischen Abschnitts 50 sowie des zylindrischen Abschnitts 52 des Grundkörpers 14. Im dritten Abschnitt 102 weist die Außenkante 36 der Finne 26 einen Winkel 38, 40, 42 relativ zur Haupterstreckungsrichtung 16 von höchstens 5° auf. Der zweite Abschnitt 100 ist in proximal Richtung betrachtet hinter dem ersten Abschnitt 98 angeordnet. Der dritte Abschnitt 102 ist in proximal Richtung betrachtet hinter dem zweiten Abschnitt 100 angeordnet. Der zweite Abschnitt 100 verbindet den ersten Abschnitt 98 und den dritten Abschnitt 102 miteinander.

Die Finne 26 weist eine Höhe 44 relativ zum Grundkörper 14 auf. Ferner weist die Finne 26 eine Dicke 46 auf. Die Dicke 46 ist senkrecht zur Haupterstreckungsebene 34 der Finne 26 gemessen. Die Höhe 44 der Finne 26 entspricht wenigstens einer doppelten Dicke 46 der Finne 26 und zwar insbesondere im ersten Abschnitt 98 der Finne 26. Aufgrund verschiedener Abschnitte des Grundkörpers 14 und der Finne 26 nimmt eine relative Höhe 44 der Finne 26 in proximaler Richtung betrachtet relativ zum Grundkörper 14 gemessen hin ab.

Die Finne 26 ist wenigstens teilweise aus einem elastischen Material ausgebildet. Das elastische Material weist einen Shorewert von wenigstens 40 auf. Ferner weist das elastische Material einen Shorewert von höchstens 80 auf. Im vorliegenden Fall weist das elastische Material einen Shorewert von zumindest im Wesentlichen 62 auf. Bei dem Material handelt es sich um einen medizinischen Kunststoff. Das Material ist ein thermoplastisches Elastomer. Im vorliegenden Fall ist das Material unter dem Markennamen Mediprene bekannt.

Die Finne 26 ist im vorliegenden Fall einstückig mit dem Grundkörper 14 ausgebildet. Die Finne 26 ist im vorliegenden Fall aus dem gleichen Material wie der Grundkörper 14. Die Finne 26 und der Grundkörper 14 bilden eine Spritzgussbaugruppe aus. Alternativ könnten die Finnen 26, 28, 30 und der Grundkörper 14 voneinander separat hergestellt werden. Die Finne 26 und der Grundkörper 14 könnten dann miteinander verbunden werden. Beispielsweise könnten die Finne 26 und der Grundkörper 14 miteinander verschweiß oder verklebt werden. Ferner ist eine Herstellung des Grundkörpers und der Finne 26 durch einen Mehrkomponentenspritzguss denkbar.

Der Schnellverbinder 22 weist wenigstens eine weitere Finne 28 auf. Die weitere Finne 28 ist zumindest im Wesentlichen identisch zur Finne 26 ausgebildet. Im vorliegenden Fall weist der Schnellverbinder 22 wenigstens eine zusätzliche Finne 30 auf. Die zusätzliche Finne 30 ist zumindest im Wesentlichen identisch zur Finne 26 ausgebildet. Demnach weist der Schnellverbinder 22 insgesamt drei Finnen 26, 28, 30 auf. Ferner könnte der Schnellverbinder 22 eine von der hier gezeigten abweichende Anzahl an Finnen 26, 28, 30 umfassen, um beispielsweise eine Klemmkraft zu variieren.

Die Finnen 26, 28, 30 sind zueinander drehsymmetrisch an dem Grundkörper 14 angeordnet. Im vorliegenden Fall sind die Finnen 26, 28, 30 entsprechend einer dreifachen Drehsymmetrie an dem Grundkörper 14 angeordnet. Der Grad der Drehsymmetrie kann dabei über die Anzahl von Finnen varriert werden. Eine Drehsymmetrieachse 94 der Drehsymmetrie der Finnen 26, 28, 30 ist verschieden von der Drehsymmetrieachse 73. Die Drehsymmetrisachse 94 ist identisch mit der Drehsymmetrieachse 72. Die Drehsymmetrieachse 94 der Finnen 26, 28, 30 ist parallel zu der Haupterstreckungsrichtung 16 des Grundkörpers 14. Die Drehsymmetrieachse 94 der Finnen 26, 28, 30 ist parallel zur Haupterstreckungsrichtung 16 des Grundkörpers 14 versetzt.

Aufgrund der Tatsache, dass der Grundkörper 14 frei von einer Drehsymmetrie ist und die Finnen 26, 28, 30 in einer dreifachen Drehsymmetrie entsprechend angeordnet sind weist der Knickschutz 12 insgesamt selbst keine Drehsymmetrie auf. Der Knickschutz 12 weist eine Spiegelsymmetrie auf. Eine Spiegelebene der Spiegelsymmetrie des Knickschutzes 12 entspricht der Haupterstreckungsebene 34 der Finne 26.

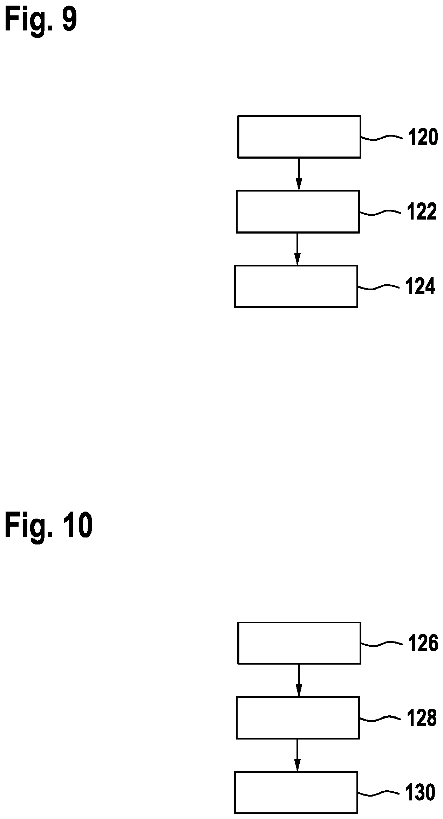

In Fig. 9 ist ein schematischer Ablaufplan eines Verfahrens zur Herstellung der endoskopischen Vorrichtung dargestellt.

Das Verfahren umfasst zumindest einen Verfahrensschritt 120. In dem Verfahrensschritt 120 wird eine Form erzeugt, welche einem Negativ des gewünschten Knickschutzes 12 entspricht.

Das Verfahren umfasst wenigstens einen weiteren Verfahrensschritt 122. In dem weiteren Verfahrensschritt 122 wird das Material verflüssigt. Das Material wird in die Form gegeben. Das Material wird in der Form ausgehärtet. Der Knickschutz 12 bildet sich aus.

Das Verfahren umfasst einen weiteren Verfahrensschritt 124. In dem weiteren Verfahrensschritt 124 wird der Knickschutz 12 aus der Form entnommen. Daraufhin ist der Knickschutz 12 einsatzbereit.

In Fig 10 ist eine schematischer Ablaufplan eines beispielhaften Verfahrens zur Montage und/oder Demontage des Knickschutz 12 dargestellt.

Das Verfahren umfasst wenigstens einen Verfahrensschritt 126. In dem Verfahrensschritt 126 wird der Endoskopschaft 10 in die Ausnehmung 74 des Knickschutzes 12 eingeführt. Der Knickschutz 12 wird in proximal Richtung entlang dem Endoskopschaft 10 geführt. Der Knickschutz 12 wird bis zum Erreichen der Handhabe 62 entlang dem Endoskopschaft 10 geführt. Ferner wird der Knickschutz 12 mit der Handhabe 62 verbunden.

Das Verfahren umfasst wenigstens einen weiteren Verfahrensschritt 128. In dem weiteren Verfahrensschritt 128 wird der Tubus 20 auf den Endoskopschaft 10 geführt. Der Tubus 20 wird auf den Endoskopschaft 10 geführt, bis der proximale Endabschnitt des Tubus 20 mit dem Knickschutz 12 in Kontakt kommt.

Das Verfahren umfasst wenigstens einen weiteren Verfahrensschritt 130. In dem weiteren Verfahrensschritt 130 wird der Tubus 20 mittels des Schnellverbinders 22 mit dem Knickschutz 12 verbunden. Der proximale Endabschnitt 92 des Tubus 20 wird über die Finnen 26, 28, 30 des Knickschutzes 12 geführt. Dabei drückt der proximale Endabschnitt 92 des Tubus 20 die Finnen 26, 28, 30 zur Seite, sodass sich diese seitlich verformen und innerhalb des Endabschnitts verklemmen. Die Finne 26, 28, 30 verklemmen den Tubus 20 von Innen mit dem Knickschutz 12.

Ferner kann der Tubus 22 demontiert werden, indem der Tubus 20 in distaler Richtung mit einer Kraft beaufschlagt wird, welche größer ist als die den Finnen 26, 28, 30 auf den Tubus 20 wirkende Klemmkraft. Alternativ oder zusätzlich kann der Tubus 20 relativ zum Knickschutz 12 gedreht werden, um derart eine Haftreibung der Finnen 26, 28, 30 seitlich zu überwinden und so eine auf die Innenwand des Tubus wirkende Klemmkraft zu reduzieren.

| | | | |
|---|---|---|---|
| 10 | Endoskopschaft | 60 | Endoskop |
| 12 | Knickschutz | 62 | Handhabe |
| 14 | Grundkörper | 64 | Handgriff |
| 16 | Haupterstreckungsrichtung | 66 | Gehäuse |
| 18 | Haupterstreckung | 68 | Bedienelement |
| 20 | Tubus | 70 | Kanal |
| 22 | Schnellverbinder | 72 | Drehsymmetrieachse |
| 26 | Finne | 73 | Drehsymmetrieachse |
| 28 | Finne | 74 | Ausnehmung |
| 30 | Finne | 76 | Mantel |
| 32 | Erstreckung | 78 | Außenfläche |
| 34 | Haupterstreckungsebene | 80 | Innenfläche |
| 36 | Außenkante | 82 | Wandung |
| 38 | Winkel | 84 | Verbindungselement |
| 40 | Winkel | 86 | Korrespondierendes Verbindungselement |
| 42 | Winkel | | |
| 44 | Höhe | 88 | Arretierelement |
| 46 | Dicke | 90 | Arretierelement |
| 48 | Abschnitt | 92 | Endabschnitt |
| 50 | Abschnitt | 94 | Drehsymmetrieachse |
| 52 | Abschnitt | 98 | Abschnitt |
| 54 | Querschnitt | 100 | Abschnitt |
| 56 | Querschnitt | 102 | Abschnitt |
| 58 | Querschnitt | 120 | Verfahrensschritt |
| | | 122 | Verfahrensschritt |
| 124 | Verfahrensschritt | 128 | Verfahrensschritt |
| 126 | Verfahrensschritt | 130 | Verfahrensschritt |

## Patentansprüche

1. Endoskopische Vorrichtung mit wenigstens einem flexiblen Endoskopschaft (10) und mit wenigstens einem dem Endoskopschaft (10) zugeordneten Knickschutz (12), welcher wenigstens einen, zumindest abschnittsweise konisch und/oder zylindrisch ausgebildeten Grundkörper (14), der eine Haupterstreckungsrichtung (16) sowie eine Haupterstreckung (18) entlang der Haupterstreckungsrichtung (16) aufweist, umfasst, und der Knickschutz (12) wenigstens einen zu einer wahlweisen Verbindung mit einem Tubus (20) eingerichteten Schnellverbinder (22) umfasst, welcher wenigstens eine, zumindest im Wesentlichen senkrecht auf dem Grundkörper (14) stehende Finne (26, 28, 30) aufweist, **dadurch gekennzeichnet, dass** die Finne (26, 28, 30) eine Erstreckung (32) aufweist, welche sich entlang der Haupterstreckungsrichtung (16) des Grundkörpers (14) wenigstens über 50 % der Haupterstreckung (18) des Grundkörpers (14) erstreckt.

2. Endoskopische Vorrichtung nach Anspruch 1, **dadurch gekennzeichnet, dass** eine Haupterstreckungsebene (34) der Finne (26, 28, 30) zumindest im Wesentlichen parallel zu Haupterstreckungsrichtung (16) des Grundkörpers (14) ausgerichtet ist.

3. Endoskopische Vorrichtung nach einem der vorhergehenden Ansprüche, **dadurch gekennzeichnet, dass** die Finne (26, 28, 30) wenigstens eine dem Grundkörper (14) abgewandte Außenkante (36) aufweist, wobei wenigstens ein Winkel (38, 40, 42) der Außenkante (36) relativ zur Haupterstreckungsrichtung (16) des Grundkörpers (14) entlang dessen Haupterstreckung (18) abnimmt.

4. Endoskopische Vorrichtung nach einem der vorhergehenden Ansprüche, **dadurch gekennzeichnet, dass** die Finne (26, 28, 30) dazu eingerichtet ist, sich bei einer Kontaktierung, insbesondere mit dem Tubus (20), seitlich zu verbiegen.

5. Endoskopische Vorrichtung nach einem der vorhergehenden Ansprüche, **dadurch gekennzeichnet, dass** die Finne (26, 28, 30) wenigstens teilweise elastisch ausgebildet ist.

6. Endoskopische Vorrichtung nach einem der vorhergehenden Ansprüche, **dadurch gekennzeichnet, dass** die Finne (26, 28, 30) wenigstens teilweise aus einem Material besteht, welches einen Shorewert von wenigstens 40 und/oder höchstens 80 aufweist.

7. Endoskopische Vorrichtung nach einem der vorhergehenden Ansprüche, **dadurch gekennzeichnet, dass** die Finne (26, 28, 30) wenigstens teilweise aus einem Material besteht, bei welchem es sich um ein thermoplastisches Elastomer handelt.

8. Endoskopische Vorrichtung nach einem der vorhergehenden Ansprüche, **dadurch gekennzeichnet, dass** die Finne (26, 28, 30) wenigstens relativ zum Grundkörper (14) gemessen abschnittsweise eine Höhe (44) aufweist, welche wenigstens einer doppelten Dicke (46) der Finne (26, 28, 30) entspricht.

9. Endoskopische Vorrichtung nach einem der vorhergehenden Ansprüche, **dadurch gekennzeichnet, dass** der Grundkörper (14) zumindest einen konischen Abschnitt (48, 52) und wenigstens einen zylindrischen Abschnitt (50) aufweist.

10. Endoskopische Vorrichtung nach einem der vorhergehenden Ansprüche, **dadurch gekennzeichnet, dass** der Grundkörper (14) senkrecht zu dessen Haupterstreckungsrichtung (16) geschnitten, wenigstens abschnittsweise einen von einer Kreisringförmigen Form abweichenden Querschnitt (54, 56) aufweist.

11. Endoskopische Vorrichtung nach einem der vorhergehenden Ansprüche, **dadurch gekennzeichnet, dass** die Finne (26, 28, 30) einstückig mit dem Grundkörper (14) ausgebildet ist.

12. Endoskopische Vorrichtung nach einem der vorhergehenden Ansprüche, **dadurch gekennzeichnet, dass** der Schnellverbinder (22) wenigstens eine weitere Finne (26, 28, 30) aufweist, welche im Wesentlichen identisch zu der Finne (26, 28, 30) ausgebildet ist.

13. Endoskopische Vorrichtung nach Anspruch 12, **dadurch gekennzeichnet, dass** die Finne (26, 28, 30) und die weitere Finne (26, 28, 30) um eine Drehsymmetrieachse (72, 94) relativ zueinander drehsymmetrisch an dem Grundkörper (14) angeordnet sind.

14. Endoskopische Vorrichtung nach Anspruch 13, **dadurch gekennzeichnet, dass** die Drehsymmetrieachse (94) der Finnen (26, 28, 30) verschieden ist von der Haupterstreckungsrichtung (16) des Grundkörpers (14).

15. Endoskopische Vorrichtung nach einem der vorhergehenden Ansprüche, **gekennzeichnet durch** den Tubus (20).

16. Endoskop (60) mit einer endoskopischen Vorrichtung mit wenigstens einem flexiblen Endoskopschaft (10) und mit wenigstens einem dem Endoskopschaft (10) zugeordneten Knickschutz (12), welcher wenigstens einen, zumindest abschnittsweise konisch und/oder zylindrisch ausgebildeten Grundkörper (14), der eine Haupterstreckungsrichtung (16) sowie eine Haupterstreckung (18) entlang der Haupterstreckungsrichtung (16) aufweist, umfasst, und der Knickschutz (12) wenigstens einen zu einer wahlweisen Verbindung mit einem Tubus (20) eingerichteten Schnellverbinder (22) umfasst, welcher wenigstens eine, zumindest im Wesentlichen senkrecht auf dem Grundkörper (14) stehende Finne (26, 28, 30) aufweist, **dadurch gekennzeichnet, dass** die Finne (26, 28, 30) eine Erstreckung (32) aufweist, welche sich entlang der Haupterstreckungsrichtung (16) des Grundkörpers (14) wenigstens über 50 % der Haupterstreckung (18) des Grundkörpers (14) erstreckt.

17. Endoskop (60) nach Anspruch 16, **dadurch gekennzeichnet, dass** die endoskopische Vorrichtung zusätzliche Merkmale nach einem der Ansprüche 2 bis 15 umfasst.

## Claims

1. An endoscopic device having at least one flexible endoscope shaft (10) and having at least one kink protector (12) assigned to the endoscope shaft (10), which comprises at least one base body (14), which is designed conically and/or cylindrically at least in sections and which has a main extension direction (16) and a main extension (18) along the main extension direction (16), and the kink protector (12) comprises at least one quick connector (22), which is configured to selectively connect to a tube (20) and which has at least one fin (26, 28, 30) situated at least substantially perpendicularly on the base body (14), **characterized in that** the fin (26, 28, 30) has an extension (32), which extends along the main extension direction (16) of the base body (14) at least over 50% of the main extension (18) of the base body (14).

2. The endoscopic device according to claim 1, **characterized in that** a main extension plane (34) of the fin (26, 28, 30) is aligned at least substantially parallel to the main extension direction (16) of the base body (14).

3. The endoscopic device according to one of the preceding claims, **characterized in that** the fin (26, 28, 30) has at least one outer edge (36) facing away from the base body (14), wherein at least one angle (38, 40, 42) of the outer edge (36) relative to the main extension direction (16) of the base body (14) decreases along its main extension (18).

4. The endoscopic device according to one of the preceding claims, **characterized in that** the fin (26, 28, 30) is configured to bend laterally upon contact, in particular with the tube (20).

5. The endoscopic device according to one of the preceding claims, **characterized in that** the fin (26, 28, 30) is designed to be at least partially elastic.

6. The endoscopic device according to one of the preceding claims, **characterized in that** the fin (26, 28, 30) consists at least partially of a material which has a Shore value of at least 40 and/or at most 80.

7. The endoscopic device according to one of the preceding claims, **characterized in that** the fin (26, 28, 30) consists at least partially of a material which is a thermoplastic elastomer.

8. The endoscopic device according to one of the preceding claims, **characterized in that** the fin (26, 28, 30) has, in sections and measured at least relative to the base body (14), a height (44) which corresponds to at least double the thickness (46) of the fin (26, 28, 30).

9. The endoscopic device according to one of the preceding claims, **characterized in that** the base body (14) has at least one conical section (48, 52) and at least one cylindrical section (50).

10. The endoscopic device according to one of the preceding claims, **characterized in that** the base body (14) has, sectioned perpendicular to its main extension direction (16), at least in sections a cross-section (54, 56) deviating from a circular ring shape.

11. The endoscopic device according to one of the preceding claims, **characterized in that** the fin (26, 28, 30) is designed in one piece with the base body (14).

12. The endoscopic device according to one of the preceding claims, **characterized in that** the quick connector (22) has at least one further fin (26, 28, 30), which is designed substantially identically to the fin (26, 28, 30).

13. The endoscopic device according to claim 12, **characterized in that** the fin (26, 28, 30) and the further fin (26, 28, 30) are arranged on the base body (14) so as to be rotationally-symmetric relative to one another about a rotational symmetry axis (72, 94).

14. The endoscopic device according to claim 13, **characterized in that** the rotational symmetry axis (94) of the fins (26, 28, 30) is different from the main extension direction (16) of the base body (14).

15. The endoscopic device according to one of the preceding claims, **characterized by** the tube (20).

16. An endoscope (60) comprising an endoscopic device having at least one flexible endoscope shaft (10) and having at least one kink protector (12) assigned to the endoscope shaft (10), which comprises at least one base body (14), which is designed conically and/or cylindrically at least in sections and which has a main extension direction (16) and a main extension (18) along the main extension direction (16), and the kink protector (12) comprises at least one quick connector (22), which is configured to selectively connect to a tube (20) and which has at least one fin (26, 28, 30) situated at least substantially perpendicularly on the base body (14), **characterized in that** the fin (26, 28, 30) has an extension (32), which extends along the main extension direction (16) of the base body (14) at least over 50% of the main extension (18) of the base body (14).

17. The endoscope (60) according to claim 16, **characterized in that** the endoscopic device comprises additional features according to one of claims 2 to 15.

## Revendications

1. Dispositif endoscopique ayant au moins une tige d'endoscope flexible (10) et ayant au moins une protection contre pliages (12) associée à la tige d'endoscope (10), qui comprend au moins un corps de base (14), qui est conçu de manière conique et/ou cylindrique au moins par sections et qui a une direction d'extension principale (16) et une extension principale (18) le long de la direction d'extension principale (16), et le protecteur contre pliages (12) comprend au moins un raccord rapide (22), qui est configuré pour se raccorder sélectivement à un tube (20) et qui a au moins une ailette (26, 28, 30) située au moins sensiblement perpendiculaire au corps de base (14), **caractérisé en ce que** l'ailette (26, 28, 30) a une extension (32), qui s'étend le long de la direction d'extension principale (16) du corps de base (14) au moins sur 50 % de l'extension principale (18) du corps de base (14).

2. Dispositif endoscopique selon la revendication 1, **caractérisé en ce qu'un** plan d'extension principal (34) de l'ailette (26, 28, 30) est aligné au moins sensiblement parallèle à la direction d'extension principale (16) du corps de base (14).

3. Dispositif endoscopique selon l'une des revendications précédentes, **caractérisé en ce que** l'ailette (26, 28, 30) a au moins un bord extérieur (36) orienté à l'opposé du corps de base (14), dans lequel au moins un angle (38, 40, 42) du bord extérieur (36) par rapport à la direction d'extension principale (16) du corps de base (14) diminue le long de son extension principale (18).

4. Dispositif endoscopique selon l'une des revendications précédentes, **caractérisé en ce que** l'ailette (26, 28, 30) est configurée pour fléchir latéralement au contact, en particulier avec le tube (20).

5. Dispositif endoscopique selon l'une des revendications précédentes, **caractérisé en ce que** l'ailette (26, 28, 30) est conçue pour être au moins partiellement élastique.

6. Dispositif endoscopique selon l'une des revendications précédentes, **caractérisé en ce que** l'ailette (26, 28, 30) est constituée au moins partiellement d'un matériau qui a une valeur Shore d'au moins 40 et/ou d'au plus 80.

7. Dispositif endoscopique selon l'une des revendications précédentes, **caractérisé en ce que** l'ailette (26, 28, 30) est constituée au moins partiellement d'un matériau qui est un élastomère thermoplastique.

8. Dispositif endoscopique selon l'une des revendications précédentes, **caractérisé en ce que** l'ailette (26, 28, 30) a, par sections et mesurée au moins par rapport au corps de base (14), une hauteur (44) qui correspond au moins au double de l'épaisseur (46) de l'ailette (26, 28, 30).

9. Dispositif endoscopique selon l'une des revendications précédentes, **caractérisé en ce que** le corps de base (14) a au moins une section conique (48, 52) et au moins une section cylindrique (50).

10. Dispositif endoscopique selon l'une des revendications précédentes, **caractérisé en ce que** le corps de base (14) a, coupé perpendiculairement à sa direction principale d'extension (16), au moins par sections, une section transversale (54, 56) s'écartant d'une forme d'anneau circulaire.

11. Dispositif endoscopique selon l'une des revendications précédentes, **caractérisé en ce que** l'ailette (26, 28, 30) est conçue d'un seul tenant avec le corps de base (14).

12. Dispositif endoscopique selon l'une des revendications précédentes, **caractérisé en ce que** le raccord rapide (22) a au moins une ailette (26, 28, 30) supplémentaire, qui est conçue de manière sensiblement identique à l'ailette (26, 28, 30).

13. Dispositif endoscopique selon la revendication 12, **caractérisé en ce que** l'ailette (26, 28, 30) et l'ailette (26, 28, 30) supplémentaire sont agencées sur le corps de base (14) de manière à être symétriques en rotation l'une par rapport à l'autre autour d'un axe de symétrie de rotation (72, 94).

14. Dispositif endoscopique selon la revendication 13, **caractérisé en ce que** l'axe de symétrie de rotation (94) des ailettes (26, 28, 30) est différent de la direction d'extension principale (16) du corps de base (14).

15. Dispositif endoscopique selon l'une des revendications précédentes, **caractérisé par** le tube (20).

16. Endoscope (60) comprenant un dispositif endoscopique ayant au moins une tige d'endoscope flexible (10) et ayant au moins un protecteur contre pliages (12) associé à la tige d'endoscope (10), qui comprend au moins un corps de base (14), qui est conçu de manière conique et/ou cylindrique au moins par sections et qui a une direction d'extension principale (16) et une extension principale (18) le long de la direction d'extension principale (16), et le protecteur contre pliages (12) comprend au moins un raccord rapide (22), qui est configuré pour se raccorder sélectivement à un tube (20) et qui a au moins une ailette (26, 28, 30) située au moins sensiblement perpendiculaire au corps de base (14), **caractérisé en ce que** l'ailette (26, 28, 30) a une extension (32), qui s'étend le long de la direction d'extension principale (16) du corps de base (14) au moins sur 50 % de l'extension principale (18) du corps de base (14).

17. Endoscope (60) selon la revendication 16, **caractérisé en ce que** le dispositif endoscopique comprend des caractéristiques supplémentaires selon l'une des revendications 2 à 15.
